# EUROPEAN PATENT APPLICATION

(11) **EP 2 253 303 A2**
(43) Date of publication of application: **24.11.2010**
(21) Application number: 10163005.1
(22) Date of filing: 17.05.2010
(51) Int. Cl.: A61K 8/04, A61K 8/25, A61Q 1/06, A61K 8/891, A61K 8/58, A61K 8/92

(54) **Comfortable, transfert-resistant colored compositions**

(30) Priority: 18.05.2009 US 179191 P
(71) Applicant: L'Oréal, 75008 Paris (FR)
(72) Inventor: Ashini, Amin, Monroe, NJ NJ 08831 (US); Rohmeyer, Ann Marie, Englishtown, NJ NJ 07726 (US); Carter, Shirley, Middlesex, NJ NJ 08846 (US)
(74) Representative: Le Coupanec, Pascale A.M.P.

(57) **Abstract**

A composition for application to the skin includes (a) at least one film forming resin chosen from a polypropylsilsesquioxane wax substituted with alkyl units having at least 30 carbon atoms; (b) at least one volatile solvent capable of solubilizing (a); (c) at least one gelling agent chosen from a modified clay; and (d) a structuring agent, wherein the composition has a hardness value ranging from about 85g to about 145g.

## Description

### BACKGROUND OF THE INVENTION

Compositions used to make up a user's skin must be able to impart color with little or no transfer. They must also provide good wear properties. The transfer resistance and wear of compositions are usually obtained through the use of film forming resins such as silicone film forming resins. While the use of silicone film forming resins in colored cosmetics is popular, one drawback associated with their use is that they tend to be brittle and flake off. This phenomenon results in the need to use a plasticizer, in combination with the resin, in order to render the resultant film more flexible and, hence, less susceptible to flake off and poor transfer resistance. Moreover, the resultant films formed by the resins are uncomfortable on human skin.

Therefore, it is an object of the present invention to provide a method and compositions for making up skin in a manner which delivers a combination of transfer resistance, superior comfort and feel. It is also an object of the present invention to provide compositions which are stable.

### BRIEF SUMMARY OF THE INVENTION

In one embodiment of the present invention, there are provided compositions for application to the skin, comprising:(a) at least one film forming resin chosen from a polypropylsilsesquioxane wax substituted with alkyl units having at least 30 carbon atoms; (b) at least one volatile solvent capable of solubilizing (a); (c) at least one gelling agent chosen from a modified clay; and (d) at least one structuring agent, wherein the compositions have a hardness value ranging from about 85g to about 145g.

Another aspect of the present invention is directed to a method of making up skin involving applying onto the skin the above-disclosed compositions.

It has been surprisingly discovered that the above-described compositions are stable, have a unique texture, and provide transfer resistance and superior comfort.

### DETAILED DESCRIPTION OF THE INVENTION

Other than in the operating examples, or where otherwise indicated, all numbers expressing quantities of ingredients and/or reaction conditions are to be understood as being modified in all instances by the term "about", meaning within 10% to 15% of the indicated number.

### FILM FORMING RESINS

The compositions of the present invention comprise at least one film-forming resin chosen from a polypropylsilsesquioxane wax substituted with alkyl units having at least 30 carbon atoms. Polypropylsilsesquioxane waxes, in general, have been disclosed in patent publication WO2005/100444, hereby incorporated by reference in its entirety.

It should be noted that not all polypropylsilsesquioxane waxes yield stable colored cosmetic emulsion products. More particularly, it has been found that only those polypropylsilsesquioxane waxes substituted with alkyl units having at least 30 carbon atoms are stable.

The polypropylsilsesquioxane wax comprises at least 40 mole % of siloxy units having the formula (R₂R'SiO_{1/2})ₓ(C₃H₇SiO_{3/2})_{y}, where x and y have a value of 0.05 to 0.95, R is an alkyl group having from 1 to 8 carbon atoms, and R' is a monovalent hydrocarbon having 30 to 40 carbon atoms and greater. As used herein, x and y represent the mole fraction of (R₂R'SiO_{1/2}) and (C₃H₇SiO_{3/2}) siloxy units relative to each other present in the polypropylsilsesquioxane wax. Thus, the mole fraction of (R₂R'SiO_{1/2}) and (C₃H₇SiO_{3/2}) siloxy units each can independently vary from 0.05 to 0.95. Preferably R is a methyl, and R' is an alkyl having at least 30 carbons, available from Dow Corning.

Typically, the value of x is 0.05 to 0.95, or alternatively, 0.2 to 0.8, the value of y is 0.05 to 0.95, alternatively 0.2 to 0.8. However, the combination of (R₂R'SiO_{1/2}) and (C₃H₇SiO_{3/2}) siloxy units present must total at least 40 mole %; alternatively 60 mole %; or alternatively 90 mole % of all siloxy units present in the polypropylsilsesquioxane wax.

The number average molecular weight of the polypropylsilsesquioxane wax substituted with alkyl units having at least 30 carbon atoms typically ranges from about 750 to about 10,000, such as from about 1,000 to about 5,000.

A suitable example of polypropylsilsesquioxane resin for use in cosmetic compositions of the present invention includes, but is not limited to, a polypropylsilsesquioxane resin commercially available from Dow-Corning under the tradename DC 670 Fluid.

The polypropylsilsesquioxane wax substituted with alkyl units having at least 30 carbon atoms is generally present in the compositions of the present invention in an amount ranging from about 1% to about 25% by weight, such as from about 5% to about 20% by weight, all weights being based on the weight of the compositions as a whole.

### SOLVENTS

The compositions of the present invention also contain at least one volatile solvent capable of solubilizing the polypropylsilsesquioxane wax substituted with alkyl units having at least 30 carbon atoms. Examples of suitable volatile solvents include, but are not limited to, volatile silicone oils and volatile non-silicone oils.

Suitable volatile silicone oils include, but are not limited to, linear or cyclic silicone oils having a viscosity at room temperature less than or equal to 6cSt and having from 2 to 7 silicon atoms, these silicones being optionally substituted with alkyl or alkoxy groups of 1 to 10 carbon atoms. Specific oils that may be used in the invention include octamethyltetrasiloxane, decamethylcyclopentasiloxane, dodecamethylcyclohexasiloxane, heptamethyloctyltrisiloxane, hexamethyldisiloxane, decamethyltetrasiloxane, dodecamethylpentasiloxane and their mixtures. Other volatile oils which may be used include KF 96A of 6 cSt viscosity, a commercial product from Shin Etsu having a flash point of 94°C. Preferably, the volatile silicone oils have a flash point of at least 40°C.

Non-limiting examples of volatile silicone oils are listed in Table 1 below.

**Table 1**

| Compound | Flash Point | Viscosity |
|---|---|---|
| | (°C) | (cSt) |
| Octyltrimethicone | 93 | 1.2 |
| Hexyltrimethicone | 79 | 1.2 |
| Decamethylcyclopentasiloxane (cyclopentasiloxane or D5) | 72 | 4.2 |
| Octamethylcyclotetrasiloxane (cyclotetradimethylsiloxane or D4) | 55 | 2.5 |
| Dodecamethylcyclohexasiloxane (D6) | 93 | 7 |
| Decamethyltetrasiloxane(L4) | 63 | 1.7 |
| KF-96 A from Shin Etsu | 94 | 6 |
| PDMS (polydimethylsiloxane) DC 200 (1.5cSt) from Dow Corning | 56 | 1.5 |
| PDMS DC 200 (2cSt) from Dow Corning | 87 | 2 |
| PDMS DC 200 (5cSt) from Dow Corning | 134 | 5 |
| PDMS DC 200 (3St) from Dow Corning | 102 | 3 |

Preferably the volatile solvent capable of solubilizing the polypropylsilsesquioxane resin comprises cyclopentasiloxane.

Suitable volatile non-silicone oils may be selected from volatile hydrocarbon oils, alcohols, volatile esters and volatile ethers. Examples of such volatile non-silicone oils include, but are not limited to, volatile hydrocarbon oils having from 8 to 16 carbon atoms and their mixtures and in particular branched C₈ to C₁₆ alkanes such as C₈ to C₁₆ isoalkanes (also known as isoparaffins), isododecane, isodecane, isohexadecane, and for example, the oils sold under the trade names of Isopar or Permethyl, the C₈ to C₁₆ branched esters such as isohexyl or isodecyl neopentanoate and their mixtures. Preferably, the volatile non-silicone oils have a flash point of at least 40°C.

Non-limiting examples of volatile non-silicone oils are listed in Table 2 below.

**Table 2**

| Compound | Flash Point (°C) |
|---|---|
| Isododecane | 43 |
| Isohexadecane | 102 |
| Isodecyl Neopentanoate | 118 |
| Propylene glycol n-butyl ether | 60 |
| Ethyl 3-ethoxypropionate | 58 |
| Propylene glycol methylether acetate | 46 |
| Isopar L (isoparaffin C₁₁-C₁₃) | 62 |
| Isopar H (isoparaffin C₁₁-C₁₂) | 56 |

The at least one volatile solvent is generally present in the compositions of the present invention in an amount ranging from about 5% to about 60% by weight; such as from about 10% to about 40% by weight; such as from about 15% to about 30% by weight, all weights being based on the weight of the compositions as a whole.

It may be desirable to also employ a non-volatile solvent in the compositions of the present invention.

Examples of non-volatile sovlents that may be used in the present invention include non-volatile silicone oils such as linear polydimethylsiloxanes (PDMSs), that are liquid at room temperature; polydimethylsiloxanes comprising alkyl, alkoxy or phenyl groups, which are pendent and/or at the end of a silicone chain, these groups each containing from 2 to 24 carbon atoms; phenylsilicones, for instance phenyl trimethicones, phenyl dimethicones, phenyl trimethylsiloxydiphenylsiloxanes, diphenyl dimethicones, diphenyl methyldiphenyl trisiloxanes, 2-phenylethyl trimethylsiloxysilicates, trimethyl pentaphenyl trisiloxane, tetramethyl hexaphenyl trisiloxane.

Examples of other non-volatile oils which may be used in the compositions of the present invention include polar oils such as:

- hydrocarbon-based plant oils with a high triglyceride content consisting of fatty acid esters of glycerol, the fatty acids of which may have varied chain lengths, these chains possibly being linear or branched, and saturated or unsaturated; these oils are especially wheat germ oil, corn oil, sunflower oil, karite butter, castor oil, sweet almond oil, macadamia oil, apricot oil, soybean oil, rapeseed oil, cottonseed oil, alfalfa oil, poppy oil, pumpkin oil, sesame seed oil, marrow oil, avocado oil, hazelnut oil, grape seed oil, blackcurrant seed oil, evening primrose oil, millet oil, barley oil, quinoa oil, olive oil, rye oil, safflower oil, candlenut oil, passion flower oil or musk rose oil; or caprylic/capric acid triglycerides, for instance those sold by the company Stearineries Dubois or those sold under the names Miglyol 810, 812 and 818 by the company Dynamit Nobel;

- synthetic oils or esters of formula R₅COOR₆ in which R₅ represents a linear or branched higher fatty acid residue containing from 1 to 40 carbon atoms, including from 7 to 19 carbon atoms, and R₆ represents a branched hydrocarbon-based chain containing from 1 to 40 carbon atoms, including from 3 to 20 carbon atoms, with R₆ + R₇ ≥ 10, such as, for example, Purcellin oil (cetostearyl octanoate), isononyl isononanoate, C₁₂ to C₁₅ alkyl benzoate, isopropyl myristate, 2-ethylhexyl palmitate, and octanoates, decanoates or ricinoleates of alcohols or of polyalcohols; hydroxylated esters, for instance isostearyl lactate or diisostearyl malate; and pentaerythritol esters;

- synthetic ethers containing from 10 to 40 carbon atoms;

- C₈ to C₂₆ fatty alcohols, for instance oleyl alcohol; and

- mixtures thereof.

The non-volatile solvents, if present, will generally be used in an amount of from about 0.1 to about 35% by weight, such as from about 5 to about 20% by weight, all weights being based on the total weight of the composition.

### GELLING AGENTS

The compositions of the invention also contain at least one gelling agent chosen from a modified clay. The gelling agent increases the viscosity and leads to a solid or flowable composition. The gelling agent does not encompass waxes, in the sense that it is not waxy. The at least one gelling agent may be chosen from gelling agents in polymeric form and gelling agents in mineral form. The gelling agent may be chosen from agents that gel via chemical cross-linking and agents that gel via physical cross-linking.

Examples of suitable modified clays include, but are not limited to, hectorites modified with an ammonium chloride of a C₁₀ to C₂₂ fatty acid, such as hectorite modified with distearyldimethylammonium chloride, also known as quaternium-18 bentonite, such as the products sold or made under the names Bentone 34 by the company Rheox, Claytone XL, Claytone 34 and Claytone 40 sold or made by the company Southern Clay, the modified clays known under the name quaternium-18 benzalkonium bentonites and sold or made under the names Claytone HT, Claytone GR and Claytone PS by the company Southern Clay, the clays modified with stearyldimethylbenzoylammonium chloride, known as stearalkonium bentonites, such as the products sold or made under the names Claytone APA and Claytone AF by the company Southern Clay, and Baragel 24 sold or made by the company Rheox.

The at least one modified clay is preferably disteardimonium hectorite.

The at least one modified clay, is present in the compositions of the invention in an amount ranging from about 0.1% to about 10%; such as from about 0.1% to about 5%; and such as from about 0.1% to about 1.5%; all weights based on the weight of the compositions as a whole.

It may also be desirable to employ other types of gelling agents in the compositions of the present invention. Mineral gelling agents, which can be used in the invention, include silica, such as fumed silica. The fumed silica may have a particle size, which may be nanometric to micrometric, for example ranging from 5 nm to 200 nm.

The fumed silicas may be obtained by high-temperature hydrolysis of a volatile silicon compound in a hydrogen-oxygen flame, producing a finely divided silica. This process makes it possible to obtain hydrophilic silicas that have a large number of silanol groups at their surface. Such hydrophilic silicas are sold or made, for example, under the names "Aerosil 130^{®}", "Aerosil 200^{®}", "Aerosil 255^{®}", "Aerosil 300^{®}" and "Aerosil 380^{®}" by the company Degussa, and "CAB-O-SIL HS-5^{®}", "CAB-O-SIL EH-5^{®}", "CAB-O-SIL LM-130^{®}", "CAB-O-SIL MS-55^{®}" and "CAB-O-SIL M-5^{®}" by the company Cabot.

It is thus possible to chemically modify the surface of the hydrophilic silica by chemical reaction, producing a reduction in the number of silanol groups. The silanol groups can be replaced, for example, with hydrophobic groups: this then gives a hydrophobic silica. The hydrophobic groups may be: trimethylsiloxyl groups, which are obtained in particular by treating fumed silica in the presence of hexamethyldisilazane. Silicas thus treated are known as "silica silylate" according to the CTFA dictionary (6th edition, 1995). They are sold or made, for example, under the references "Aerosil R812^{®}" by the company Degussa and "CAB-O-SIL TS-530^{®}" by the company Cabot; dimethylsilyloxyl or polydimethylsiloxane groups, which are obtained in particular by treating fumed silica in the presence of polydimethylsiloxane or dimethyldichlorosilane. Silicas thus treated are known as "silica dimethyl silylate" according to the CTFA dictionary (6th edition, 1995). They are sold or made, for example, under the references "Aerosil R972^{®}" and "Aerosil R974^{®}" by the company Degussa, and "CAB-O-SIL TS-610^{®}" and "CAB-O-SIL TS-720^{®}" by the company Cabot; groups derived from reacting fumed silica with silane alkoxides or siloxanes. These treated silicas are, for example, the products sold or made under the reference "Aerosil R805^{®}" by the company Degussa.

According to the invention, hydrophobic silica, such as fumed silica, may be used as a lipophilic gelling agent. The use of fumed silica makes it possible to obtain a translucent or even transparent composition, in particular in the form of a stick, which does not exude, in the absence of opacifying particles such as waxes, fillers and pigments (including nacres).

The at least one lipophilic gelling agent can allow the exudation of the composition to be limited and can allow its stability to be increased, while at the same time conserving the composition's glossy appearance, which is not possible with waxes such as those used conventionally in cosmetics and dermatology.

In the event that a gelling agent, other than a modified clay, is employed, it will typically be present in the compositions of the invention in an amount ranging from about 0.1% to about 10%; such as from about 0.1% to about 5%; and such as from about 0.1% to about 2%; all weights based on the weight of the compositions as a whole.

### STRUCTURING AGENTS

The compositions of the present invention also include one or more structuring agents capable of imparting onto the compositions of the present invention a hardness valueranging from about 85g to about 145g.

The hardness value is determined by a TA-XT2i texture analyzer from Texture Technologies Corp of Scarsdale, NY. The parameters include the following:

Pre-Speed = 2.0mm/s

Test Speed = 2.0mm/s

Pre-Test Speed = 2.0mm/s

Test Distance = 2.0 mm

Trigger Force = 5 g

TA Option = Return Start

TA Mode = Measure Force in Compression

Feel and wear properties, it has been surprisingly found, are directly related to the hardness of the compositions themselves, as measured using the above referenced texture analyzer.

Examples of suitable structuring agents include but are not limited to waxes having a melting point ranging from about 60°C to about 75°C. Two such preferred waxes are candelilla wax and ceresin wax.

In a preferred embodiment, a combination of ceresin wax and candelilla wax are used. The ceresin wax is typically employed in an amount ranging from about 0.1% to about 20%; such as from about 7% to about 15% and from about 10% to about 11%. Similarly, the candelilla wax is typically employed in an amount ranging from about 0.1% to about 5%; such as from about 0.1% to about 2% and from about 0.1% to about 0.5%.

The compositions of the present invention include a combination of waxes such that the compositions have a hardness value, obtained from penetrating readings, ranging from about 85g to about 145g; such as from about 85g to about 115g.

In order to obtain the above referenced hardness values, the ceresin and candelilla waxes are typically present in the composition in a ratio by weight of from about 4:1, such as about 7:1, and such as from about 10:1.

### AUXILIARY INGREDIENTS

The compositions of the present invention may also include at least one auxiliary ingredient chosen from colorants, co-film formers, plasticizers, active ingredients such as sunscreen actives, anti-aging actives, and the like, co-solvents, fillers, preservatives, etc.

Preferably the compositions of the present invention are capable of being hot poured at a temperature of 85°C or less.

### EXAMPLES

The present invention is further described in terms of the following non-limiting examples. Unless otherwise indicated, all parts and percentages are on a weight-by-weight percentage basis.

Two cosmetic compositions were prepared containing the below-disclosed ingredients.

Example 1

| **INCI Name** | **%** |
|---|---|
| | |
| Polypropylsilsesquioxane wax⁽¹⁾& Cyclopentasiloxane | 25.30 |
| Disteardimonium Hectorite | 1.10 |
| Propylene Carbonate | 0.33 |
| Cyclopentasiloxane | 4.97 |
| Cyclohexasiloxane | 9.35 |
| Caprylyl Methicone | 5.00 |
| Ceresin wax | 10.70 |
| Candelilla wax | 0.30 |
| Black Iron Oxide & Disodium Stearoyl Glutamate & Aluminum Hydroxide | 31.00 |
| Silica Silylate | 2.00 |
| Caprylyl Glycol | 0.30 |
| Isododecane | 9.65 |
| Total | **100.00** |
| Hardness⁽²⁾ | 88.51g |

| | |
|---|---|
| (1)DOW CORNING DC 670 (2) Average reading based on 10 samples. | |

### Example 2

| **INCI Name** | **%** |
|---|---|
| | |
| Polypropylsilsesquioxane⁽¹⁾ & Cyclopentasiloxane | 25.30 |
| Disteardimonium Hectorite | 1.10 |
| Propylene Carbonate | 0.33 |
| Cyclopentasiloxane | 4.98 |
| Cyclohexasiloxane | 9.35 |
| Caprylyl Methicone | 4.99 |
| Ceresin wax | 10.70 |
| Candelilla wax | 0.30 |
| Black Iron Oxide & Disodium Stearoyl Glutamate & Aluminum Hydroxide | 10.20 |
| Ultramarines & Disodium Stearoyl Glutamate & Aluminum Hydroxide | 10.80 |
| MICA (and) TITANIUM DIOXIDE (and) FERRIC FERROCYANIDE | 10.00 |
| Silica Silylate | 2.00 |
| Caprylyl Glycol | 0.30 |
| Isododecane | 9.65 |
| | **100.00** |
| | |
| Hardness⁽²⁾ | 92.18g |

Procedure:
1. Pre-made gel phase by combining disteardimonium hectorite, cyclohexasiloxane, and caprylyl methicone. Mixed for 30 minutes.
2. Added propylene carbonate and mixed. Maintained 65° C to 70° C for optimal consistency.
3. Combined dry pigments and cyclohexasiloxane, cyclopentasiloxane and caprylyl methicone.
4. Melted ceresin and candelilla wax for pre-melted wax phase.
5. Combined dry pigments and cyclohexasiloxane, cyclopentasiloxane, caprylyl methicone with cyclopentasiloxane, cyclohexasiloxane, caprylyl methicone, polypropylsilsesquioxane and cyclopentasiloxane and heated to 80° C, mixing until homogeneous.
6. Added gel phase and mixed for 10 minutes.
7. Added pre-melted wax phase to gel phase and mixed for 5 minutes.
8. Added pearls, if any, silica silylate and mixed for 5 minutes.
9. Added caprylyl glycol and mixed for 3 minutes.
10. Added isododecane and mixed for 5 minutes.
11. Poured at a temperature of 80°C.

The inventive compositions were found to be stable, transfer resistant, comfortable and smooth feeling.

It is to be understood that the foregoing describes preferred embodiments of the invention and that modifications may be made therein without departing from the spirit or scope of the invention as set forth in the claims.

## Claims

1. A composition for application to the skin, comprising:
(a) at least one film forming resin chosen from a polypropylsilsesquioxane wax substituted with alkyl units having at least 30 carbon atoms;
(b) at least one volatile solvent capable of solubilizing (a);
(c) at least one gelling agent chosen from a modified clay; and
(d) at least one structuring agent, wherein the composition has a hardness value ranging from about 85g to about 145g.

2. The composition of claim 1, wherein (a) is present in the composition in an amount ranging from about 1% to about 25% by weight, based on the weight of the composition as a whole.

3. The composition according to any one of claims 1 to 2, wherein (a) is present in the composition in an amount ranging from about 5% to about 20%, based on the weight of the composition as a whole.

4. The composition according to any one of claims 1 to 3, wherein (b) comprises cyclopentasiloxane.

5. The composition according to any one of claims 1 to 4, wherein (b) is present in the composition in an amount ranging from about 5% to about 60% by weight, based on the weight of the composition as a whole.

6. The composition according to any one of claims 1 to 5, wherein (b) is present in the composition in an amount ranging from about 10% to about 40% by weight, based on the weight of the composition as a whole.

7. The composition according to any one of claims 1 to 6, wherein (c) comprises disteardimonium hectorite.

8. The composition according to any one of claims 1 to 7, wherein (c) is present in the composition in an amount ranging from about 0.1% to about 10% by weight, based on the weight of the composition as a whole.

9. The composition according to any one of claims 1 to 8, wherein (c) is present in the composition in an amount ranging from about 0.1% to about 5% by weight, based on the weight of the composition as a whole.

10. The composition of claim according to any one of claims 1 to 9, wherein (d) comprises at least one wax having a melting point of between about 60°C and about 75°C.

11. The composition according to any one of claims 1 to 10, wherein (d) is chosen from ceresin wax, candelilla wax and mixtures thereof.

12. The composition of claim 11, wherein the ceresin wax and candelilla wax are employed in a ratio by weight of about 4:1.

13. The composition of claim 11, wherein the ceresin wax and candelilla wax are employed in a ratio by weight of about 10:1.

14. The composition according to any one of claims 1 to 13, wherein the compositions are capable of being hot poured at a temperature of 85°C or less.

15. A method of making up skin comprising applying onto the skin a composition according to any one of claims 1 to 14.
